# EUROPEAN PATENT APPLICATION

(11) **EP 3 032 246 A1**
(43) Date of publication of application: **15.06.2016**
(21) Application number: 15821013.8
(22) Date of filing: 21.04.2015
(51) Int. Cl.: G01N 27/26, A61B 1/12, G01N 27/06

(54) **ELECTRODE-TYPE SOLUTION MEASUREMENT DEVICE, STORAGE CONTAINER, AND ENDOSCOPE REPROCESSOR**

(30) Priority: 04.09.2014 JP 2014180587
(71) Applicant: Olympus Corporation, Hachioji-shi Tokyo 192-8507 (JP)
(72) Inventor: ONISHI, Hideto, Tokyo 192-8507 (JP); KOMIYA, Takaaki, Tokyo 192-8507 (JP); AKAHORI, Hiromasa, Tokyo 192-8507 (JP)
(74) Representative: Gunzelmann, Rainer
(86) International application number: PCT/JP2015/062111
(87) International publication number: WO 2016/035377

(57) **Abstract**

In an electrode unit 80, a working electrode 88 and a counter electrode 90 are disposed in a cylindrical section 83 having a cylindrical shape, which is a sensor main body, a distal end face of the working electrode 88 is covered with a diaphragm 91 that allows liquid to be measure to permeate, and an internal liquid chamber 92 in which internal liquid of an electrolyte is stored and a standard solution chamber 93 in which a standard solution Lc for calibration is stored are partitioned by the diaphragm 91. The standard solution chamber 93 is surrounded by an inner wall of the cylindrical section 83 and a cap 84. When the electrode unit 80 is mounted on a medical solution tank 70, zero adjustment is automatically performed using a standard solution inside the electrode unit 80. Thereafter, when the cap 84 is opened, the standard solution is discharged and measurement in an actual use can be performed.

## Description

### Technical Field

The present invention relates to an electrode-type solution measuring apparatus, a storage container, and an endoscope reprocessor that can automatically perform calibration before measurement.

### Background Art

An endoscope in a medical field inserted into a body and used for the purpose of a test and treatment needs to be cleaned and disinfected after use to be used again. Therefore, it is well-known that the used endoscope is automatically cleaned and disinfected by an endoscope cleaning/disinfecting apparatus.

As a medical solution for disinfection used by the endoscope cleaning/disinfecting apparatus, the medical solution in a sealed medical solution bottle is diluted to a specified concentration with tap water or the like, stored in a medical solution tank, and used for a cleaning/disinfecting process. In this case, concentration control for the medical solution in the medical solution tank is important. Concentration of the medical solution needs to be periodically checked. However, there is a problem that complicated work such as collection and quantification of samples is necessary. Therefore, Japanese Patent Application Laid-Open Publication No. H11-64280 discloses a measuring apparatus that can perform highly accurate acid concentration measurement in a short time without weighing and collecting a liquid to be measured.

When concentration of a solution is measured by an electrode-type sensor disclosed in Japanese Patent Application Laid-Open Publication No. H11-64280, the sensor needs to be periodically replaced because deterioration of an electrode affects a measurement result. However, individual differences such as manufacturing errors are present in the electrode-type sensor and the measuring apparatus that processes a sensor signal. Therefore, measurement in an actual use is possible only when calibration processing such as zero point adjustment is performed every time the replacement is performed.

There has been a problem that, for calibration work for the sensor, preparation of a standard solution for calibration, a measurement instrument, and the like is necessary and measurement work itself for the calibration takes time.

The present invention has been devised in view of the circumstances and it is an object of the present invention to provide an electrode-type solution measuring apparatus, a storage container, and an endoscope reprocessor capable of, simply by performing replacement of an electrode unit, automatically performing calibration to realize a measurement state in an actual use.

### Disclosure of Invention

### Means for Solving the Problem

An electrode-type solution measuring apparatus according to an aspect of the present invention is an electrode-type solution measuring apparatus including an electrode unit detachably attachable to a storage container that stores liquid to be measured, which is a measurement target. The electrode unit includes: a cylindrical section having, for example, a cylindrical shape detachably inserted into the storage container; a cap that openably closes a distal end portion of the cylindrical section with stress application; a diaphragm disposed a predetermined distance apart from the cap, the diaphragm selectively allowing the liquid to be measured to permeate; a standard solution chamber surrounded by an inner wall of the cylindrical section, the cap, and the diaphragm, the standard solution chamber storing a standard solution for calibration; a working electrode disposed in the cylindrical section to be set in contact with the diaphragm; a first electrode pad electrically connected to the working electrode and disposed on a surface of the cylindrical section; an internal liquid chamber partitioned from the standard solution chamber by the diaphragm in the cylindrical section, the internal liquid chamber storing internal liquid of an electrolyte; a counter electrode disposed to be separated from the working electrode in the cylindrical section, at least a part of the counter electrode being exposed in the internal liquid chamber to come into contact with the internal liquid; and a second electrode pad electrically connected to the counter electrode and disposed on the surface of the cylindrical section.

An electrode-type solution measuring apparatus according to another aspect of the present invention is an electrode-type solution measuring apparatus including an electrode unit detachably attachable to a storage container that stores liquid to be measured, which is a measurement target. The electrode unit includes: a cylindrical section having, for example, a cylindrical shape detachably inserted into the storage container; a cap that openably closes a distal end portion of the cylindrical section with stress application; a standard solution chamber surrounded by an inner wall of the cylindrical section and the cap, the standard solution chamber storing a standard solution for calibration; a working electrode disposed in the cylindrical section, at least a part of the working electrode being exposed in the standard solution chamber to come into contact with the standard solution; a first electrode pad electrically connected to the working electrode and disposed on a surface of the cylindrical section; a counter electrode disposed to be separated from the working electrode in the cylindrical section, at least a part of the counter electrode being exposed in the standard solution chamber to come into contact with the standard solution; and a second electrode pad electrically connected to the counter electrode and disposed on the surface of the cylindrical section.

A storage container according to an aspect of the present invention is the storage container in the aspect explained above. The storage container includes: a container section for storing a medical solution; a housing section formed in a part of the container section, having a concave shape for housing the electrode unit in the aspect explained above, and having an opening in a bottom surface; a first contact exposed on an inner surface of the housing section and for electrically coming into contact with the first electrode pad of the electrode unit advanced to a first position of the housing section; and a second contact exposed on the inner surface of the housing section and for electrically coming into contact with the second electrode pad of the electrode unit advanced to the first position.

An endoscope reprocessor according to an aspect of the present invention includes the storage container in the aspect explained above.

### Brief Description of the Drawings

Fig. 1 is a perspective view showing an endoscope cleaning apparatus according to a first embodiment of the present invention and applied with the present invention;
Fig. 2 is an explanatory diagram showing an example of an internal configuration of an endoscope reprocessor according to the first embodiment;
Fig. 3 is a configuration diagram of an electrode unit for concentration measurement according to the first embodiment;
Fig. 4 is an explanatory diagram showing an electrode-unit attaching section and a sensor control section of a medical solution tank according to the first embodiment;
Fig. 5 is a flowchart of zero-point adjustment processing according to the first embodiment;
Fig. 6 is a flowchart of abnormality diagnosis processing according to the first embodiment;
Fig. 7 is an explanatory diagram showing a mounting position during zero point adjustment of the electrode unit according to the first embodiment;
Fig. 8 is an explanatory diagram showing a mounting position during concentration measurement of the electrode unit according to the first embodiment; and
Fig. 9 is a configuration diagram of an electrode unit for conductivity measurement according to a second embodiment of the present invention.

### Best Mode for Carrying Out the Invention

Embodiments of the present invention are explained below with reference to the drawings.

### [First Embodiment]

A first embodiment of the present invention indicates application of an electrode-type solution measuring apparatus to concentration measurement for measuring concentration of a solution. An endoscope reprocessor including a medical solution tank, which is a storage container, is applied. More specifically, an example is explained in which a medical solution used in an endoscope reprocessor that automatically reproduces a used endoscope or endoscope accessories is set as liquid to be measured, which is a measurement target, and concentration of the medical solution is measured.

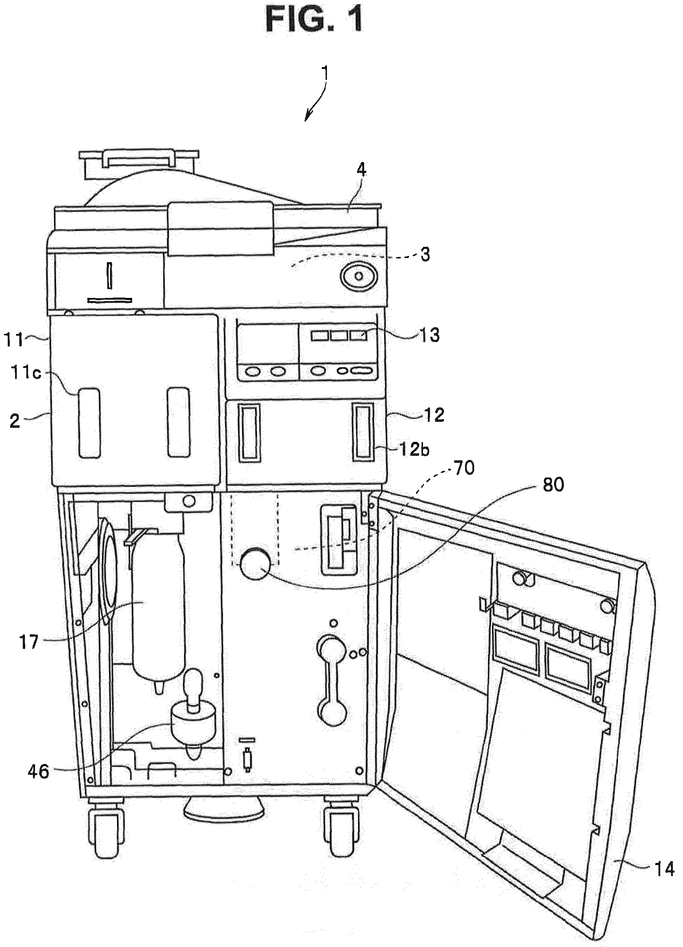

As shown in Fig. 1, an endoscope reprocessor 1 includes an apparatus main body 2 and a top cover 4 openably and closably connected to an upper part of the apparatus main body 2 via, for example, a not-shown hinge and functioning as a lid body of a treatment tank 3 that houses an endoscope. In the apparatus main body 2, a medical solution tray 11 is disposed to be capable of being drawn out to a front of the apparatus main body 2 on a front surface in Fig. 1 viewed from a front and, for example, in an upper part of a left half. A medical solution tray 12 is disposed to be capable of being drawn out to the front of the apparatus main body 2 on the front surface in Fig. 1 viewed from the front and, for example, in an upper part of a right half. An operation panel 13 for performing display of a cleaning/disinfection time, warming of the medical solution, selection of cleaning/disinfection modes, and the like may be disposed on the front surface of the apparatus main body 2 and in an upper part of the medical solution tray 12.

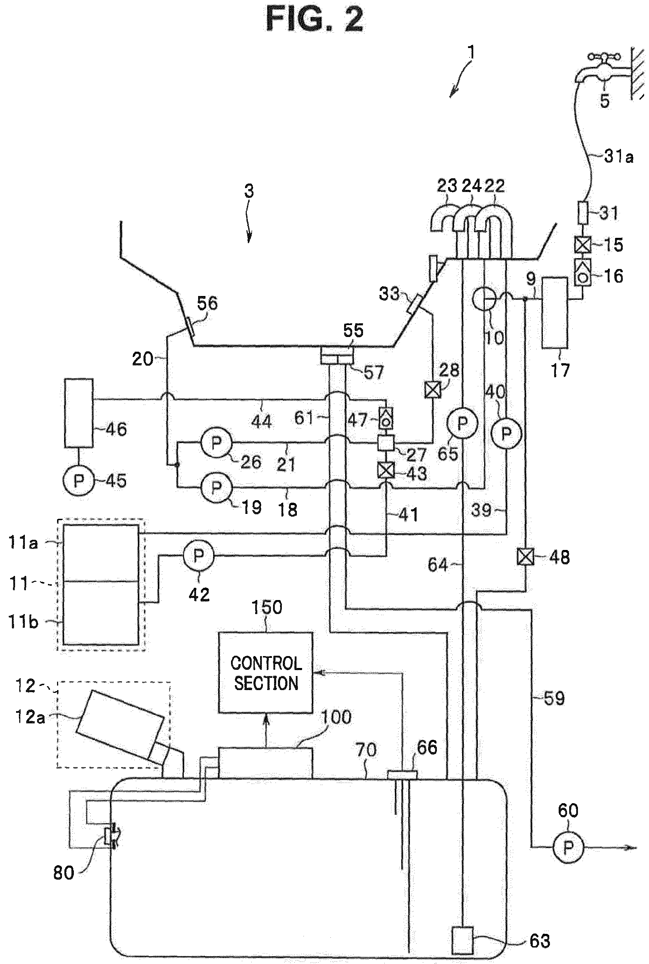

In the medical solution tray 11, a detergent tank 11a (see Fig. 2) in which a cleaning agent, which is liquid used in cleaning the endoscope, is stored and an alcohol tank 11b (see Fig. 2) in which alcohol, which is liquid used in drying the endoscope after cleaning/disinfection, is stored may be housed. Since the medical solution tray 11 is capable of being drawn out, the respective tanks are configured to be capable of being filled with the liquid. Note that an indicator 11c is provided in the medical solution tray 11. An operator can check a residual amount of the cleaning agent injected into the detergent tank and a residual amount of the alcohol injected into the alcohol tank.

On the other hand, in the medical solution tray 12, for example, a medical solution bottle 12a (see Fig. 2), in which liquid used in disinfecting the endoscope is injected, is housed. However, a medical solution stored in the medical solution tray 12 is not limited to the disinfection liquid. Cleaning liquid, rinsing liquid, or liquid having high volatility for endoscope drying may be stored. Examples of the medical solution include a solution containing peracetic acid.

The medical solution tray 12 may be configured to be capable of being drawn out such that the operator can set the medical solution bottle 12a on the medical solution tray 12 and replace the medical solution bottle 12a. Note that check windows 12b are provided in the medical solution tray 12. The operator can check, through these check windows 12b, a residual amount of the medical solution injected into the medical solution bottle 12a.

A front door 14 may be openably and closably disposed between a center part and a lower part of the front surface in the figure of the apparatus main body 2. As shown in Fig. 1, for example, in a closed state, the operator performs operation for depressing a predetermined portion of the front door 14, whereby a lock state is released and the front door 14 can be opened.

On the front surface in Fig. 1 viewed from the front of the apparatus main body 2 in a state in which the front door 14 is opened and, for example, in a right half, a side portion side of a medical solution tank 70 (see Fig. 2), which is a storage container for storing liquid to be measured that is a measurement target of concentration measurement in the present embodiment, is exposed. In an upper part of the side portion side of the medical solution tank 70, an electrode unit 80 for measuring concentration of a medical solution in the medical solution tank 70 is detachably attached. On the front surface in Fig. 1 viewed from the front of the apparatus main body 2 in the state in which the front door 14 is opened, for example, in a lower part of a left half, an air filter 46, which is a filter for air feeding is disposed. A water supply filter 17 for tap water supplied from an outside is disposed above the air filter 46.

Next, an example of an internal configuration of the endoscope reprocessor 1 is schematically explained with reference to Fig. 2. As shown in Fig. 2, the endoscope reprocessor 1 includes a configuration in which one end of a water supply hose 31a is connected to a water-supply-hose connection port 31 and the other end of the water supply hose 31a is connected to a tap water faucet 5 on an outside, whereby tap water is supplied. The water-supply-hose connection port 31 is caused to communicate with a water supply conduit 9 via a water-supply electromagnetic valve 15, a check valve 16, and the water supply filter 17. The water supply conduit 9 is connected to a three-way electromagnetic valve 10. The water supply conduit 9 may be connected to an electromagnetic valve for dilution 48 that continues and discontinues supply of dilution water to the medical solution tank 70.

Note that the water supply filter 17 can be configured as a filtration filter of a cartridge type such that the water supply filter 17 can be periodically replaced. The water supply filter 17 removes foreign matters, bacteria, and the like of tap water that passes through the water supply filter 17.

The three-way electromagnetic valve 10 is connected to one end of a flowing liquid conduit 18. The three-way electromagnetic valve 10 switches, with a valve on an inside, communication of the water supply conduit 9 and the flowing liquid conduit 18 with a water-supply circulation nozzle 24 disposed in the treatment tank 3. That is, the water-supply circulation nozzle 24 communicates with one of the water supply conduit 9 and the flowing liquid conduit 18 according to a switching operation of the three-way electromagnetic valve 10. On the other end side of the flowing liquid conduit 18, a flowing liquid pump 19, which is a pump of a non-self-priming type that can transfer only liquid and is excellent in a transfer ability of the liquid, may be disposed.

A circulation port 56 is disposed on a bottom side of the treatment tank 3. One end of a circulation conduit 20 is connected to the circulation port 56. The other end of the circulation conduit 20 branches into two to communicate with the other end of the flowing liquid conduit 18 and one end of a channel conduit 21. The other end of the channel conduit 21 communicates with respective ports for air/water feeding/forceps 33. Although not shown in the figure, the other end of the channel conduit 21 may communicate with a not-shown port for forceps raising.

In the channel conduit 21, a channel pump 26, a channel block 27, and a channel electromagnetic valve 28 are respectively disposed in order from the one end side halfway in the conduit. The channel pump 26 may be configured with a self-priming type pump that can transfer both of liquid and gas at higher pressure than the non-self-priming type pump.

A detergent nozzle 22 can also be disposed in the treatment tank 3. One end of a cleaning agent conduit 39 is connected to the detergent nozzle 22. The other end of the cleaning agent conduit 39 is connected to the detergent tank 11a of the medical solution tray 11. In the cleaning agent conduit 39, a pump for detergent 40 configured with a high-pressure self-priming type pump for raising the cleaning agent from the detergent tank 11a to the treatment tank 3 is disposed halfway in the cleaning agent conduit 39.

An alcohol tank 11b can also be disposed in the medical solution tray 11. The alcohol tank 11b is connected to one end of an alcohol conduit 41. The alcohol conduit 41 is connected to the channel block 27 to communicate with the channel conduit 21 as predetermined. In the alcohol conduit 41, an alcohol supply pump 42 configured with a high-pressure self-priming type pump for raising alcohol from the alcohol tank 11b to the treatment tank 3 and an electromagnetic valve 43 are disposed.

One end of an air conduit 44 for supplying air from an air pump 45 configured with a self-priming type pump, which can transfer air, is connected to the channel block 27 to communicate with the channel conduit 21 as predetermined. The other end of the air conduit 44 is connected to the air pump 45. A check valve 47 and the air filter 46, which is periodically replaced, are disposed in halfway positions of the air conduit 44.

A drain port 55 is provided in a bottom surface section of the treatment tank 3. An open-closable switching valve 57 for discharging the cleaning liquid and the like to the outside and collecting the medical solution in the medical solution tank 70 according to a switching operation of the valve is disposed in a lower part of the drain port 55. The switching valve 57 is connected to one end of a drain conduit 59, the other end of which is connected to and communicates with a not-shown drain hose connected to an external drain port. In the drain conduit 59, a drain pump 60 configured with a non-self-priming type pump is disposed. The switching valve 57 is connected to one end of a medical solution collection conduit 61. The other end of the medical solution collection conduit 61 is connected to the medical solution tank 70.

For example, disinfection liquid is supplied to the medical solution tank 70 from the medical solution bottle 12a as the medical solution. The disinfection liquid is used for a disinfection process after being diluted to a specified concentration. In the medical solution tank 70, one end portion of a medical solution conduit 64, at one end of which a suction filter 63 is provided, is housed as predetermined. The other end of the medical solution conduit 64 is connected to a medical solution nozzle 23 disposed in the treatment tank 3. A medical solution pump 65 configured with a high-pressure self-priming type pump for raising the disinfection liquid from the medical solution tank 70 to the treatment tank 3 is disposed in a halfway position of the medical solution conduit 64.

The dilution of the medical solution in the medical solution tank 70 is performed on the basis of a detection signal from a water level sensor 66 provided in the medical solution tank 70. The water level sensor 66 is configured to be capable of detecting, for example, a plurality of water levels including an initial water level of the dilution water supplied into the medical solution tank 70, a water level of the disinfection water obtained by diluting a disinfection medical solution with the dilution water, and an abnormal water level. A detection signal of the water level sensor 66 is inputted to a control section 150 that controls an operation of the entire endoscope reprocessor 1.

First, the control section 150 detects, with a signal of a not-shown switch or the like, detachment of the medical solution bottle 12a from the disinfection medical solution tray 12. When it is detected that the medical solution bottle 12a is attached anew, the control section 150 opens the electromagnetic valve for dilution 48 to supply the dilution water to the medical solution tank 70, supplies the dilution water to the medical solution tank 70 until the dilution water reaches a water level set in advance, and compounds a medical solution having the specified concentration.

As another control method, a method explained below can also be adopted.

First, when detecting, with a signal of the not-shown switch or the like, detachment of the medical solution bottle 12a from the disinfection medical solution tray 12, the control section 150 opens the electromagnetic valve for dilution 48 to supply the dilution water to the medical solution tank 70 and supplies a water level set in advance (a water level detected lowest) as the initial water level. When it is detected that the medical solution bottle 12a is attached anew after the dilution water reaches the initial water level or before the dilution water reaches the initial water level, the control section 150 opens the electromagnetic valve for dilution 48 again or as it is to supply the dilution water to the medical solution tank 70, supplies the dilution water to the medical solution tank 70 until the dilution water reaches a water level set in advance higher than the initial water level, and compounds a medical solution having the specified concentration.

Concentration of a medical solution stored in the medical solution tank 70 is measured using the electrode unit 80. When it is confirmed by the concentration measurement performed using the electrode unit 80 that the medical solution in the medical solution tank 70 has the specified concentration, the medical solution pump 65 is driven under control by the control section 150 and the medical solution is supplied into the treatment tank 3. The control section 150 performs driving control for the respective pumps, the respective electromagnetic valves, and the like explained above.

In the driving control for the respective valves and the respective pumps, the control section 150 includes a water-supply-conduit disinfection program for performing known drainage, disinfection, and a rinse in at least the water supply conduit 9 via the circulation conduit 20 and the channel conduit 21 and collecting, in the medical solution tank 70, at least one of the tap water and the medical solution in the treatment tank 3 via the medical solution collection conduit 61 or draining the tap water or the medical solution from the external drain port via the drain conduit 59. The control section 150 also includes an all-conduit disinfection program for disinfecting all the conduits in the endoscope reprocessor 1 and an endoscope cleaning/disinfection program for cleaning and disinfecting an endoscope conduit of an endoscope connected to the port 33 via a tube.

Next, a measurement system that measures the concentration of the medical solution stored in the medical solution tank 70 is explained. In the present embodiment, the electrode unit 80 is configured as a sensor unit for measuring the concentration of the medical solution stored in the medical solution tank 70 and as a diaphragm-type electrode unit including a diaphragm that selectively allows a medical solution, which is liquid to be measured, to permeate. More specifically, the diaphragm can cause only specific ions or molecules of the liquid to be measured to permeate. The diaphragm-type electrode unit needs to be periodically replaced in a predetermined period in order to maintain measurement accuracy. Therefore, the electrode unit 80 is detachably attached to the medical solution tank 70.

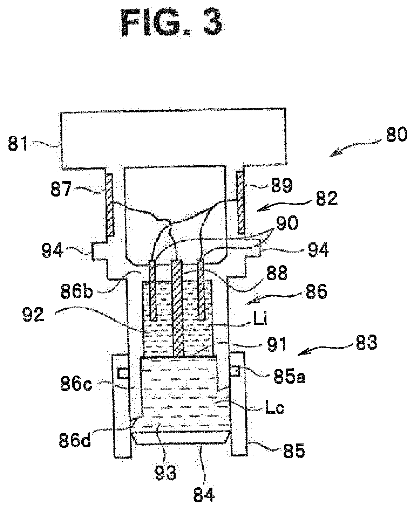

As shown in Fig. 3, the electrode unit 80 includes a flange-like set section 81 functioning as a grasping section for mounting the electrode unit 80 on the medical solution tank 70, a terminal section 82 provided below the set section 81 and on an outer circumferential surface of which first and second electrode pads 87 and 89 are disposed as terminals for external connection, a cylindrical section 83 formed below the terminal section 82 in a diameter smaller than a diameter of the terminal section 82 and including a cylindrical inner cavity, which is a sensor main body in which an electrode is disposed, and a lid-like cap 84 that openably closes a distal end side opening of the cylindrical section 83 with stress application.

The cylindrical section 83 includes a cylindrical container-like cap holding section 85 that holds the cap 84 on a distal end side and a sensor section 86 mounted on an inner circumferential surface of the cap holding section 85 to be capable of advancing and retracting. The sensor section 86 is a part formed integrally with the terminal section 82. On the other hand, the cap holding section 85 is formed separately from the sensor section 86 and mounted to cover a distal end side of the sensor section 86.

Note that the cap holding section 85 and the sensor section 86 do not always have to be configured separately. As explained below, for example, when stress is applied to the cap 84 from a mounting section side of the medical solution tank 70 to open the cap 84, the sensor section 86 does not have to be capable of advancing and retracting with respect to the cap holding section 85.

In an inner cavity center portion of the sensor section 86, a bar-like working electrode 88 electrically connected to a first electrode pad 87 disposed on an outer surface of the terminal section 82 is projected to predetermined height in a center axis direction. As explained below, the first electrode pad 87 is disposed in predetermined length in a circumferential direction to be capable of coming into contact with an electric contact on the medical solution tank 70 side in a range of a predetermined rotation angle.

In a partition wall 86b forming a bottom surface of an inner cavity of the sensor section 86 and functioning as a boundary with the terminal section 82, two bar-like counter electrodes 90 electrically connected to a second electrode pad 89 disposed on the outer surface of the terminal section 82 are separated from the working electrode 88 and erected. Distal end sides of the counter electrodes 90 are exposed in an inner cavity around an electrode holding section 96a. The second electrode pad 89 is opposed to the first electrode pad 87 and disposed in predetermined length in the circumferential direction to be capable of coming into contact with the electric contact on the medical solution tank 70 side in a range of a predetermined rotation angle.

A distal end face of the working electrode 88 is in contact with and covered by a diaphragm 91 that allows the liquid to be measured to permeate. The diaphragm 91 is formed as a circular film that seals a periphery in a position of the distal end face of the working electrode 88. An outer circumferential section of the diaphragm 91 is closely attached and fixed to a step section provided on an inner cavity wall surface of the sensor section 86.

A region surrounded by the inner cavity wall surface of the sensor section 86, an outer circumferential surface of the working electrode 88, the partition wall 86b of the bottom surface, and the diaphragm 91 is formed as an internal liquid chamber 62. In the internal liquid chamber 92, internal liquid Li is filled such that at least parts of the counter electrodes 90 come into contact with the internal liquid Li. The internal liquid Li is a solution of an electrolyte for causing the working electrode 88 and the counter electrodes 90 to electrically communicate with each other.

The outer circumferential surface of the sensor section 86 is formed as an advancing/retracting section 86c mounted in the cap holding section 85 to be capable of advancing and retracting. A distal end side of the advancing/retracting section 56c is formed as an opening section 86d for breaking and opening a part of a joining section of the cap holding section 85 and the cap 84. The opening section 86d is formed to be a sharp edge shape by, for example, cutting the distal end side of the advancing/retracting section 86c obliquely with respect to a center axis.

A region surrounded by the inner cavity wall surface of the sensor section 86 (an inner circumferential surface of the advancing/retracting section 86c), the inner circumferential surface of the cap holding section 85, the diaphragm 91, and the cap 84 is formed as a standard solution chamber 93 partitioned from the internal liquid chamber 92 by the diaphragm 91. A standard solution Lc for calibration is stored in the standard solution chamber 93. As the standard solution Lc, for example, pure water or liquid to be measured having the specified concentration is used.

Note that, on an inner circumference side of the cap holding section 85, a seal section 85a that liquid-tightly retains a space between the cap holding section 85 and the outer circumferential surface of the advancing/retracting section 86c is provided.

When the electrode unit 80 is mounted in the medical solution tank 70, the working electrode 88 and the counter electrodes 90 are electrically connected to an electric contact on the medical solution tank 70 side via the first electrode pad 87 and the second electrode pad 89 provided in the terminal section 82. At this point, after being set in a calibration position for zero point adjustment performed using the standard solution at a time of initial setting, the electrode unit 80 is set in a measurement position for an actual use after completion of the zero point adjustment. Therefore, on the electrode unit 80 side, fitting sections 94 for alignment during setting are provided in lower parts of the terminal section 82. The fitting sections 94 are formed in a protrusion shape projecting in a radial direction and are provided to be opposed to each other, for example, in predetermined two positions in the circumferential direction. The number of electrodes may be two or more. For example, two points for zero point correction and two points for measurement may be separately provided (it is conceivable to, for example, provide fixed resistance in electrodes for zero point correction for improving accuracy of the zero point correction).

Note that, as explained below, the electrode unit 80 is inserted into an attaching section of the medical solution tank 70 in two stages. At a stage when the electrode unit 80 is inserted to a first stage, the entire electrode unit 80 is rotated and pushed in, whereby the fitting sections 94 are fit in the medical solution tank 70 side and set in the measurement state for an actual use. Therefore, it is desirable to provide marks or the like indicating the positions of the fitting sections 94 on a top surface of the set section 81 and provide, on the medical solution tank 70 side, an indicator indicating a set position such as a "calibration (zero point adjustment) position" or a "measurement position (PUSH)".

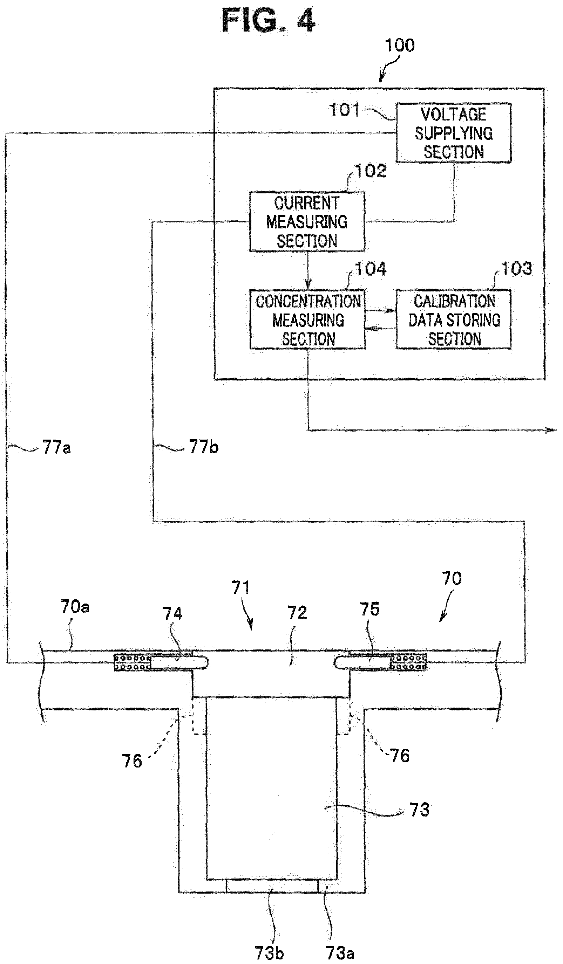

On the medical solution tank 70 side, which is a storage container, as shown in Fig. 4, a concave-shaped housing section 71 that detachably houses the electrode unit 80 is provided in a part of a container section 70a for storing the medical solution. The housing section 71 is configured with a first housing section 72 on an inlet side and a second housing section 73 formed on a depth side from the first housing section 72.

The first housing section 72 is a part that houses the terminal section 82 of the electrode unit 80 including the fitting sections 94 for alignment. A bar-like first contact 74 and a bar-like second contact 75 are opposed to each other in the radial direction and disposed such that distal end portions thereof are exposed to an inner surface side. The first contact 74 and the second contact 75 are respectively disposed to be urged to radial reaction inner sides. When the electrode unit 80 is inserted into the housing section 71, respective distal end portions of the first and second contacts 74 and 75 come into contact with the first electrode pad 87 and the second electrode pad 89, whereby electric connection is established.

The second housing section 73 is a part that is formed smaller in diameter than the first housing section 72 and houses the cylindrical section 83 of the electrode unit 80. On a bottom surface side, a contact section 73a, with which a distal end face of the cap holding section 85 of the electrode unit 80 comes into contact, is provided. On an inner side of the contact section 73a, a liquid-to-be-measured inlet 73b for guiding the liquid to be measured to the electrode unit 80 is opened.

In a step section forming an annular plane in a boundary between the first housing section 72 and the second housing section 73, measurement set sections 76 that enable the fitting sections 94 of the electrode unit 80 to move in the axial direction and specify a position during measurement are opened. The measurement set sections 76 are provided to be opposed to each other in predetermined two positions in the circumferential direction to correspond to the fitting sections 94 in the two places of the electrode unit 80. The measurement set sections 76 are opened at predetermined depth in the axial direction from the step section to an inner wall of the second housing section 73.

The first and second contacts 74 and 75 are connected to a sensor control section 100 via signal lines 77a and 77b. The sensor control section 100 is configured on a control board set in the medical solution tank 70. The sensor control section 100 includes a voltage supplying section 101 that supplies a voltage for measurement applied between the working electrode 88 and the counter electrodes 90 from the first and second contacts 74 and 75 on the medical solution tank 70 side via the first and second electrode pads 87 and 89 of the electrode unit 80, a current measuring section 102 that measures an electric current flowing between the working electrode 88 and the counter electrodes 90, a calibration data storing section 103 that stores calibration data for zero point adjustment, and a concentration measuring section 104 that corrects measurement data of a current value with the calibration data and measures concentration of the medical solution.

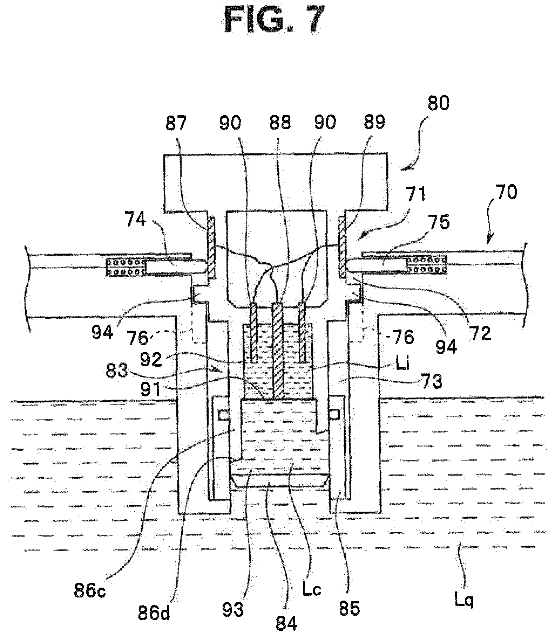

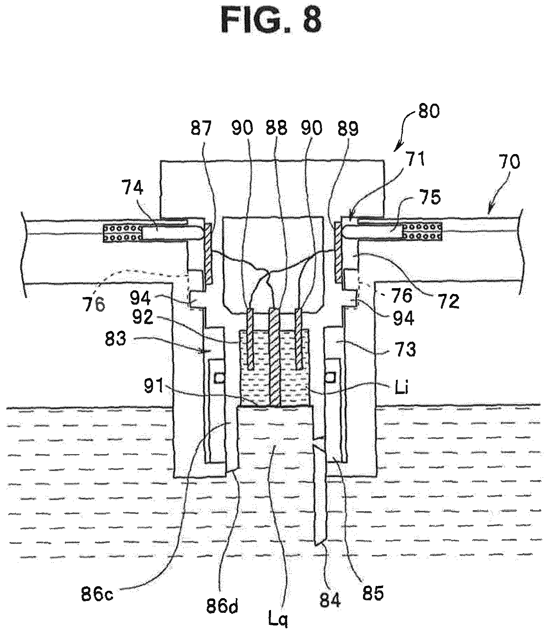

When the electrode unit 80 is set in a calibration position (see Fig. 7) in initial attachment to the medical solution tank 70 by, for example, being replaced, the sensor control section 100 performs adjustment of a reference point (a zero point) of measurement with the standard solution held by the electrode unit 80 itself and stores calibration data of the adjustment. Thereafter, when the electrode unit 80 is set in a measurement position (see Fig. 8), the sensor control section 100 measures concentration of the medical solution stored in the medical solution tank with reference to the zero point adjusted by the stored calibration data. A result of the concentration measurement of the medical solution by the sensor control section 100 is transmitted to the control section 150. The control section 150 diagnoses presence or absence of abnormality on the basis of concentration measurement data of the medical solution.

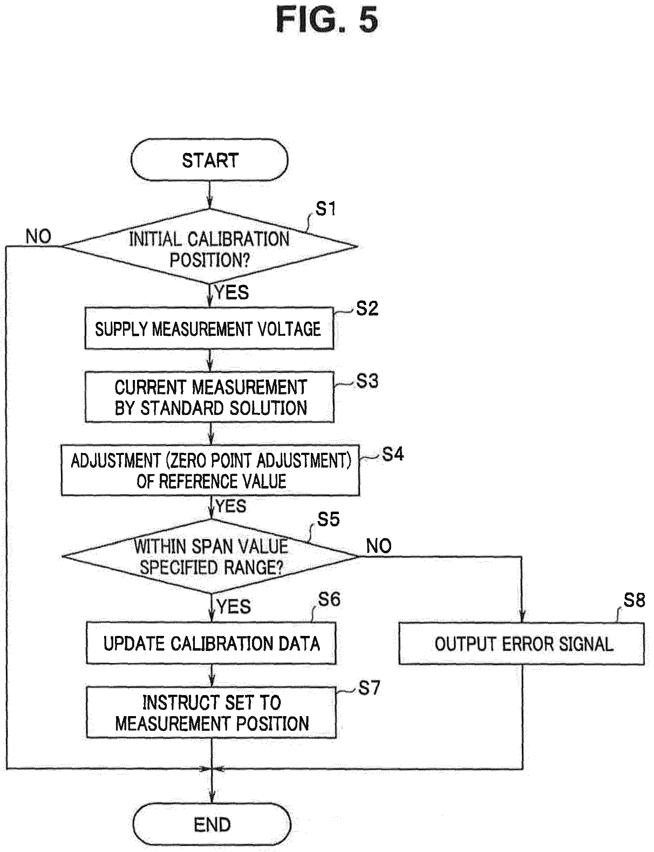

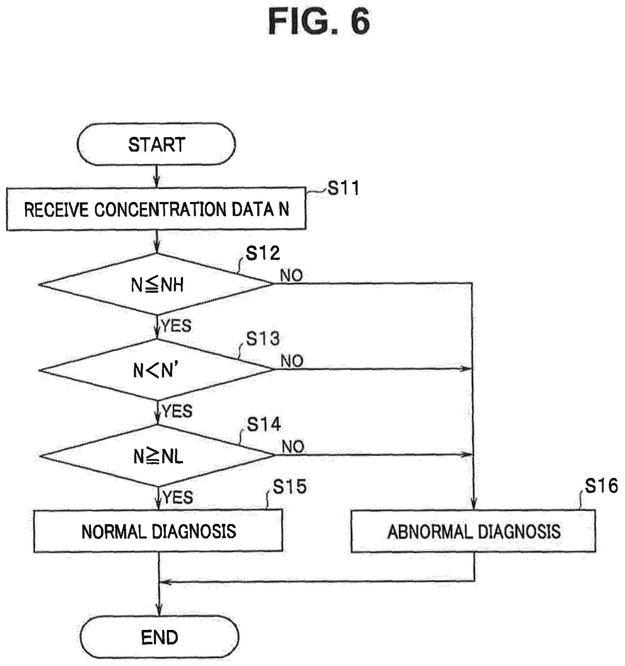

Zero-point adjustment processing of the electrode unit 80 and abnormality diagnosis processing based on the concentration measurement result of the medical solution are explained below with reference to flowcharts of Fig. 5 and Fig. 6.

First, the zero-point adjustment processing of the electrode unit 80 shown in Fig. 5 is explained. In the present embodiment, the zero-point adjustment processing is processing executed by the sensor control section 100. In first step S1, the sensor control section 100 checks whether the electrode unit 80 is set in an initial calibration position.

As shown in Fig. 7, the calibration position of the electrode unit 80 is a state in which the electrode unit 80 is inserted into the housing section 71 of the medical solution tank 70 and advances to a first position in a first stage and electric connection of the first and second contacts 74 and 75 and the first and second electrode pads 87 and 89 are established. At this point, in the electrode unit 80, the fitting sections 94 are inserted in a rotation position where fitting sections 94 do not interfere with the first and second contacts 74 and 75 and brought into contact with a step section of a boundary between the first housing section 72 and the second housing section 73. A distal end of the cylindrical section 83 is housed in the second housing section 73 while being kept closed by the cap 84.

Whether the electrode unit 80 is set in the initial calibration position can be checked by, for example, detecting an axial direction position of the electrode unit 80 using an optical or magnetic non-contact sensor, a position detection sensor configured by a contact-type limit switch, or the like. Whether the electrode unit 80 is set in the initial calibration position can also be checked by monitoring presence or absence and a history of energization between the first contact 74 and the second contact 75 on the medical solution tank 70 side.

When it is confirmed that the electrode unit 80 is set in the initial calibration position, the sensor control section 100 supplies a voltage for measurement (for adjustment) from the voltage supplying section 101 and applies a voltage between the working electrode 88 and the counter electrodes 90 from the first and second contacts 74 and 75 via the first and second electrode pads 87 and 89 (step S2). According to the voltage application, ions of the standard solution Lc for calibration stored in the standard solution chamber 93 permeates through the diaphragm 91 and a feeble current flows between the working electrode 88 and the counter electrodes 90. The electric current flowing between the working electrode 88 and the counter electrodes 90 is measured by the current measuring section 102 (step S3).

Subsequently, the concentration measuring section 104 performs, on the basis of concentration of the standard solution Lc, which is a known value in advance, adjustment of a reference value (a zero point) of concentration measurement by the measured electric current (step S4). The concentration measuring section 104 checks a zero-point correction value of measurement by the electrode unit 80 at the adjusted zero point and checks whether the zero-point correction value is within a specified range (step S5).

As a result, when the zero-point correction value is outside the specified range, a manufacturing error or the like of the electrode unit 80 is conceivable. Therefore, the sensor control section 100 outputs an error signal and transmits abnormality occurrence to the control section 150 (step S8). The control section 150 receives the error signal of the abnormality occurrence, informs the operator of the abnormality occurrence through display on the operation panel 13 or a sound output, and urges the operator to replace the electrode unit 80 with a new electrode unit 80.

On the other hand, when the zero-point correction value is within the specified range, the sensor control section 100 updates old data stored in the calibration data storing section 103 with calibration data of this time of the zero point adjustment (step S6) and transmits a signal for instructing setting of the electrode unit 80 to the measurement position to the control section 150 (step S7). The control section 150 receives the instruction for setting to the measurement position and guides, through display on the operation panel 13 or a sound output, the operator to set the electrode unit 80 in the measurement position.

The electrode unit 80 is set in the measurement position by, when the electrode unit 80 is advanced from the first position in the first stage to a second position in a second stage of the housing section 71 of the medical solution tank 70, that is, in the insertion position (the calibration position) in the first stage, rotating the electrode unit 80, matching the fitting sections 94 with the measurement set sections 76 of the medical solution tank 70, and, in that state, pushing the electrode unit 80 in a direction of an inside of the medical solution tank 70.

At this point, as shown in Fig. 8, in the cylindrical section 83 housed in the second housing section 73, the distal end face of the cap holding section 85 comes into contact with the contact section 73a at a distal end of the second housing section 73. Movement of the cap holding section 85 is restricted. The sensor section 86 (the advancing/retracting section 86c) moves relatively to the cap holding section 85. According to the relative movement of the advancing/retracting section 86c to the cap holding section 85, the opening section 86d at a distal end of the advancing/retracting section 86c breaks a part of the joining section of the cap holding section 85 and the cap 84 and opens the cap 84.

Note that the opening of the cap 84 may be performed on the medical solution tank 70 side. For example, the cap 84 may be opened by providing a part having a shape same as a shape of the opening section 86d on the bottom surface side of the second housing section 73 in an opposite direction and bumping the cap 84 against the part.

According to the opening of the cap 84, the standard solution Lc in the standard solution chamber 93 is discharged, the standard solution chamber 93 is filled with a medical solution Lq in the medical solution tank 70, and preparation for concentration measurement performed using the medical solution Lq as the liquid to be measured is completed. The concentration measurement is basically the same as the calibration by the standard solution Lc. When a voltage for measurement is supplied from the voltage supplying section 101 and a voltage is applied between the working electrode 88 and the counter electrodes 90 from the first and second contacts 74 and 75 via the first and second electrode pads 87 and 89, ions of the medical solution Lq entering the standard solution chamber 93 penetrates through the diaphragm 91. An electric current flows between the working electrode 88 and the counter electrodes 90. The electric current is measured by the current measuring section 102 and a current value is sent to the concentration measuring section 104.

After adjusting a zero point with the calibration data stored in the calibration data storing section 103, the concentration measuring section 104 calculates concentration of the medical solution Lq by, for example, referring to a data table in which a relation between concentration and a current value is recorded. A concentration measurement result of the medical solution Lq is transmitted from the concentration measuring section 104 to the control section 150. The medical solution is not limited to the disinfection liquid as explained above and may be cleaning liquid, rinsing liquid, or liquid having high volatility for endoscope drying.

The control section 150 executes the diagnosis processing shown in Fig. 6 on the basis of the concentration measurement result transmitted from the concentration measuring section 104. According to the diagnosis processing, the control section 150 diagnoses abnormality of concentration data due to a dilution failure of a medical solution due to replacement of the medical solution bottle 12a and presence or absence of abnormal concentration data due to abnormality in respective processes of cleaning and disinfection.

In the diagnosis processing shown in Fig. 6, first, the control section 150 receives concentration data N of the medical solution Lq from the sensor control section 100 (step S 11) and checks whether the concentration data N is equal to or smaller than an upper limit value NH set in advance. The upper limit value NH is upper limit allowable concentration in the case in which the medical solution in the medical solution bottle 12a is supplied into the medical solution tank 70 and diluted by water. For example, if concentration of the medical solution in the medical solution bottle 12a is 6%, immediately after the medical solution bottle 12a is replaced and the medical solution is normally diluted, the medical solution in the medical solution tank 70 is approximately 0.3%. Therefore, a diagnosis is performed with the allowable upper limit concentration Nh set to, for example, 0.35%.

As a result, when N>NH, a dilution error of the medical solution due to abnormality of a water supply system or a water level measuring system, an error of a zero-point correction value of concentration measurement, an attachment mistake of the electrode unit 80, and the like are conceivable. Therefore, the control section 150 diagnoses this as abnormality occurrence and emits a warning through display on the operation panel 13, alarm sound such as a buzzer, or voice (step S16). On the other hand, when N≤NH and the concentration data N indicates a normal value for the time being, further, the control section 150 checks whether the concentration data N of this time is higher than concentration data N' in a process of the last time (step S13).

In general, the medical solution is diluted every time the reprocessor is operated in one process. Therefore, when N≥N' and the concentration of the medical solution is high compared with the concentration during the process of the last time on the contrary, abnormality of the electrode unit 80, an error of a zero-point correction value, a conduit failure in the apparatus, and the like are conceivable. The control section 150 diagnoses this as abnormality occurrence and emits a warning in the same manner (step S16).

On the other hand, when N<N' and the concentration data N is determined as normal, further, the control section 150 checks whether the concentration data N is equal to or larger than a lower limit value NL set in advance (step S 14). The lower limit value NL is lower limit concentration with which disinfection performance can be secured when the medical solution in the medical solution bottle 12a is diluted. For example, when the concentration of the medical solution in the medical solution bottle 12a is 6%, a diagnosis is performed with the lower limit concentration NL set to 0.2%.

When the concentration data N of this time satisfies all the conditions of N≤NH, N<N', and N≥NL, the medical solution in the medical solution tank 70 is diluted to proper concentration. The control section 150 diagnoses this as normal (step S15). On the other hand, when N<NL, the control section 150 diagnoses this as abnormal and emits a warning (step S16) and urges the operator to replace the medical solution and inspect the respective sections.

In this way, in the embodiment of the present invention, simply by performing replacement of the electrode unit 80, it is possible to automatically perform calibration to realize a measurement state in an actual use. It is possible to omit time-consuming calibration work in the past and prevent the operator from inducing an adjustment mistake.

### [Second Embodiment]

Next, a second embodiment of the present invention is explained. The second embodiment indicates application to a conductivity meter that measures conductivity of liquid to be measured.

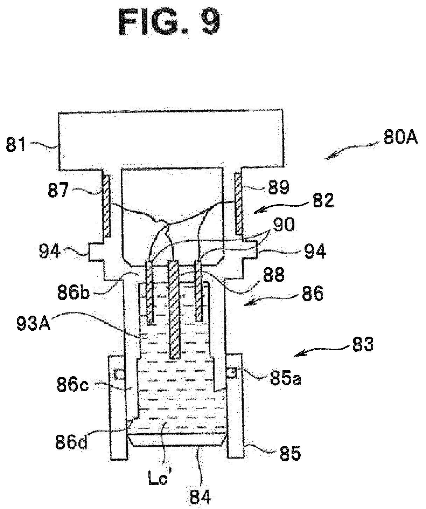

As shown in Fig. 9, in an electrode unit 80A in the second embodiment, the diaphragm 91 and the internal liquid chamber 92 of the electrode unit 80 in the first embodiment are removed to form one standard solution chamber 93A. Parts of the working electrode 88 and the counter electrodes 90 are exposed to come into contact with a standard solution Lc' for calibration stored in the standard solution chamber 93A. The standard solution Lc' for calibration is liquid having fixed conductivity. The other components are the same as the components of the electrode unit 80.

When conductivity of a solution is measured by the electrode unit 80A, an electric current flowing between the working electrode 88 and the counter electrodes 90 are measured by the standard solution Lc' in a standard solution chamber 93A in advance to perform adjustment of a zero point. Thereafter, it is possible to open the cap 84 to discharge the standard solution Lc' and measure conductivity of the liquid to be measured.

In this case, as it is well-known, the conductivity of the solution has a correlation with the concentration. The conductivity meter can be used as a concentration meter as well. Therefore, it is also possible to mount the electrode unit 80A in the medical solution tank 70 instead of the electrode unit 80 and measure concentration of a medical solution.

In the second embodiment, as in the first embodiment, simply by performing replacement of the electrode unit 80A, it is possible to automatically perform calibration to realize a measurement state in an actual use. However, in the second embodiment, it is possible to use the electrode unit 80A that is simpler compared with the electrode unit 80 in the first embodiment. It is possible to reduce system costs.

This application is based upon and claims the benefit of priority from Japanese Patent Application No. 2014-180587 filed in Japan on September 4, 2014, the contents of which are incorporated in the specification, claims, and drawings of the present application.

## Claims

1. An electrode-type solution measuring apparatus comprising an electrode unit detachably attachable to a storage container that stores liquid to be measured, which is a measurement target,
the electrode unit including:
a cylindrical section having a cylindrical shape detachably inserted into the storage container;
a cap that openably closes a distal end portion of the cylindrical section with stress application;
a diaphragm disposed a predetermined distance apart from the cap, the diaphragm selectively allowing the liquid to be measured to permeate;
a standard solution chamber surrounded by an inner wall of the cylindrical section, the cap, and the diaphragm, the standard solution chamber storing a standard solution for calibration;
a working electrode disposed in the cylindrical section to be set in contact with the diaphragm;
a first electrode pad electrically connected to the working electrode and disposed on a surface of the cylindrical section;
an internal liquid chamber partitioned from the standard solution chamber by the diaphragm in the cylindrical section, the internal liquid chamber storing internal liquid of an electrolyte;
a counter electrode disposed to be separated from the working electrode in the cylindrical section, at least a part of the counter electrode being exposed in the internal liquid chamber to come into contact with the internal liquid; and
a second electrode pad electrically connected to the counter electrode and disposed on the surface of the cylindrical section.

2. An electrode-type solution measuring apparatus comprising an electrode unit detachably attachable to a storage container that stores liquid to be measured, which is a measurement target,
the electrode unit including:
a cylindrical section having a cylindrical shape detachably inserted into the storage container;
a cap that openably closes a distal end portion of the cylindrical section with stress application;
a standard solution chamber surrounded by an inner wall of the cylindrical section and the cap, the standard solution chamber storing a standard solution for calibration;
a working electrode disposed in the cylindrical section, at least a part of the working electrode being exposed in the standard solution chamber to come into contact with the standard solution;
a first electrode pad electrically connected to the working electrode and disposed on a surface of the cylindrical section;
a counter electrode disposed to be separated from the working electrode in the cylindrical section, at least a part of the counter electrode being exposed in the standard solution chamber to come into contact with the standard solution; and
a second electrode pad electrically connected to the counter electrode and disposed on the surface of the cylindrical section.

3. The electrode-type solution measuring apparatus according to claim 1 or 2, wherein fitting sections for aligning the first electrode pad and the second electrode pad with the solution storage container are disposed on an outer surface of the cylindrical section.

4. The electrode-type solution measuring apparatus according to claim 1 or 2, wherein the cylindrical section includes:
a cap holding section that holds the cap;
an advancing/retracting section capable of advancing and retracting with respect to the cap holding section; and
an opening section provided on a distal end side of the advancing/retracting section, the opening section pushing the cap to open the cap when the advancing/retracting section advances to the cap side.

5. The storage container according to claim 1, comprising:
a container section for storing a medical solution;
a housing section formed in a part of the container section, having a concave shape for housing the electrode unit according to claim 1, and having an opening in a bottom surface;
a first contact exposed on an inner surface of the housing section and for electrically coming into contact with the first electrode pad of the electrode unit advanced to a first position of the housing section; and
a second contact exposed on the inner surface of the housing section and for electrically coming into contact with the second electrode pad of the electrode unit advanced to the first position.

6. The storage container according to claim 5, further comprising:
a voltage supplying section connected to the first contact and the second contact, the voltage supplying section supplying a voltage for measurement applied between the first contact and the second contact; and
a current measuring section connected to the first contact and the second contact, the current measuring section measuring an electric current flowing when the voltage for measurement is applied.

7. An endoscope reprocessor comprising the storage container according to claim 5 or 6.
